# EUROPÄISCHE PATENTSCHRIFT

(11) **EP 3 165 515 B1**
(45) Veröffentlichungstag und Bekanntmachung des Hinweises auf die Patenterteilung: **12.12.2018**
(21) Anmeldenummer: 16020059.8
(22) Anmeldetag: 29.02.2016
(51) Int. Cl.: C07C 1/20, C07C 5/327, C07C 11/06

(54) **VERFAHREN UND ANLAGE ZUR OLEFINSYNTHESE AUS OXYGENATEN MIT ERHÖHTER PROPYLENAUSBEUTE**
METHOD AND INSTALLATION FOR OLEFIN SYNTHESIS FROM OXYGENATES WITH INCREASED PROPYLENE YIELD
PROCÉDÉ ET INSTALLATION DE SYNTHÈSE D'OLÉFINE A PARTIR DE PRODUITS OXYGÉNÉS PRÉSENTANT UN RENDEMENT EN PROPYLÈNE ÉLEVÉ

(30) Priorität: 06.11.2015 EP 15400053
(43) Veröffentlichungstag der Anmeldung: 10.05.2017
(73) Patentinhaber: L'Air Liquide Société Anonyme pour l'Etude et l'Exploitation des Procédés Georges Claude, 75007 Paris (FR)
(72) Erfinder: Lin, Lin, 60433 Frankfurt am Main (DE); Haag, Stéphane, 60598 Frankfurt am Main (DE); Rothämel, Martin, 60437 Frankfurt/Main (DE); Castillo-Welter, Frank, 61381 Friedrichsdorf (DE); Drosdzol, Christopher, 60438 Frankfurt am Main (DE)
(74) Vertreter: Dropsch, Holger

(56) Entgegenhaltungen:
- WO-A1-2014/124844
- CN-A- 102 320 912

## Beschreibung

### Gebiet der Erfindung

Die vorliegende Erfindung betrifft ein Verfahren zur Herstellung von Olefinen, insbesondere von Propylen, aus Oxygenaten mit folgenden Schritten:
(a) heterogen-katalysierte Umsetzung eines Wasserdampf und Oxygenate, wie Methanol und/oder Dimethylether, umfassenden Eduktgemischs unter Oxygenatumsetzungsbedingungen in einem Olefinsynthesereaktor zu einem Propylen, andere Olefine, Paraffine und Aromaten enthaltenden Primärprodukt,
(b) Auftrennung des Primärprodukts mittels physikalischer Trennverfahren in eine C₅₋-Fraktion, eine C₅₊-Fraktion und eine wässrige, nicht umgesetzte Oxygenate enthaltende Phase,
(c) Auftrennung der C₅₊-Fraktion durch mehrstufige Destillation, umfassend einen Entbutaner, wobei mindestens ein Olefine enthaltender Rückführstrom erhalten wird, der zum Olefinsynthesereaktor zurückgeführt wird, und wobei ein Kohlenwasserstoffe enthaltender Benzinstrom gewonnen wird, der als Nebenprodukt aus dem Verfahren ausgeleitet wird,
(d) Verdichtung der C₅₋-Fraktion in einem ersten Verdichter und Auftrennung der verdichteten C₅₋-Fraktion in einer ersten Phasentrennstufe in ein erstes gasförmiges Verdichterprodukt und ein erstes flüssiges Verdichterprodukt,
(e) Zuführung des ersten flüssigen Verdichterprodukts zu dem Entbutaner, wobei als Kopfprodukt des Entbutaners eine C₄₋-Fraktion erhalten wird, die der verdichteten C₅₋-Fraktion zugeführt wird, und wobei das Sumpfprodukt der C₅₊-Fraktion zugeführt wird,
(f) Zuführung des ersten gasförmigen Verdichterprodukts zu einem Entpropaner, wobei als Kopfprodukt des Entpropaners eine C₃₋-Fraktion und als Sumpfprodukt eine C₄-Fraktion erhalten werden, wobei die C₄-Fraktion mindestens teilweise zum Olefinsynthesereaktor zurückgeführt wird,
(g) Zuführung der C₃₋-Fraktion zu einem Entethaner, wobei als Kopfprodukt des Entethaners eine C₂₋-Fraktion und als Sumpfprodukt eine C₃-Fraktion erhalten werden,
(h) Auftrennung der C₃-Fraktion mittels physikalischer Trennverfahren in eine Propylen umfassende Fraktion, die als Zielprodukt aus dem Verfahren ausgeleitet wird, und in eine Propan umfassende Fraktion.

Weiterhin umfasst die Erfindung eine Anlage zur Durchführung dieses Verfahrens.

### Stand der Technik

Propen (C₃H₆), oft auch als Propylen bezeichnet, ist einer der wichtigsten Ausgangsstoffe der chemischen Industrie. Die Nachfrage nach dem Grundstoff Propylen steigt weltweit, wobei Propylen ebenso wie Ethylen zumeist in einem Steam-Cracker in einem von den Verfahrensbedingungen und den Rohstoffen abhängigen Verhältnis aus Erdöl erzeugt wird.

Um zusätzliches Propylen zu gewinnen, existiert eine Reihe von Verfahren, wie etwa die Propandehydrierung (PDH), die von Propan als Edukt ausgeht. Bekannt ist jedoch vor allem das so genannte MTP-(Methanol-to-Propylene)-Verfahren, in dem sogenannte Oxygenate wie Methanol (MeOH) oder Dimethylether (DME) durch katalytische Umsetzung an einem zeolithischen Katalysator zu Olefinen umgewandelt werden. Durch Variation des Katalysators und der Prozessbedingungen kann die Selektivität der erhaltenen Produkte beeinflusst und so das Produktspektrum zu kurzkettigen Olefinen (dann oft auch die Prozessbezeichnung Methanol-to-Olefin (MTO)), zu längerkettigen Produkten (dann oft auch die Prozessbezeichnung Methanol-to-Gasoline (MTG)) oder eben zu Propylen verschoben werden.

Die Grundlagen des MTP-Verfahrens sind beispielsweise in der Offenlegungsschrift DE 10 2005 048 931 A1 beschrieben. Aus einem Wasserdampf und Oxygenate, wie Methanol und/oder Dimethylether, enthaltenden Eduktgemisch werden vor allem C₂- bis C₄-Olefine hergestellt. Dabei wird das Eduktgemisch in wenigstens einem Reaktor durch eine heterogen-katalysierte Reaktion zu einem niedermolekulare Olefine und Benzinkohlenwasserstoffe umfassenden Reaktionsgemisch umgesetzt. Durch ein geeignetes Trennkonzept können höherwertige Olefine, vor allem die C₅₊-Fraktion, als Recyclingstrom wenigstens teilweise in den Reaktor zurückgeführt und dort größtenteils zu Propylen umgewandelt werden, wodurch sich die Ausbeute an Propylen erhöht.

Das MTP-Verfahren hat üblicherweise eine Propylenausbeute von etwa 65% (mol-C). Eine erhöhte Propylenausbeute würde die Wirtschaftlichkeit des Verfahrens deutlich verbessern. Als vorherrschendes Nebenprodukt im MTP-Prozess fällt ein benzinartiges Gemisch (MTP-Benzin) an, das im Wesentlichen aus Olefinen, Paraffinen, cyclischen Kohlenwasserstoffen und Aromaten besteht. Dieses MTP-Benzin kann ebenfalls in eine sich anschließende Wertschöpfungskette eingegliedert werden, hat jedoch einen niedrigeren Marktpreis als Propylen.

Wie in der Druckschrift WO 2006/136433 A1 beschrieben, wird daher beispielsweise versucht, das MTP-Benzin bzw. Fraktionen von diesem einer Nachbearbeitung in Form einer Olefin-Metathese zu unterziehen, bei der das MTP-Benzin bzw. aus diesem gewonnene Fraktionen bei Temperaturen von etwa 400 bis 500 °C und einem Druck von 1 bis 5 bar an einem zeolithischen Katalysator umgesetzt wird. Durch diese nachgeschaltete Reaktion kann zwar eine moderate Erhöhung der Propylen-Ausbeute des Gesamtverfahrens erreicht werden, allerdings liegt die Gesamtausbeute immer noch unter 70 mol-%.

Eine direkte Rückführung des MTP-Benzins in den MTP-Reaktor bringt keine Steigerung der Ausbeute an Propylen. Da es innerhalb des MTP-Reaktors zu unerwünschten Alkylierungsreaktionen der Aromaten kommt, die Methanol verbrauchen, welches dann nicht mehr für die selektive Bildung von Propylen zur Verfügung steht, sänke die Propylenausbeute des Gesamtverfahrens bei der direkten Rückführung des MTP-Benzins in den MTP-Reaktor sogar.

Daher schlägt die internationale Patentanmeldung WO 2014/124844 A1 ein modifiziertes MTP-Verfahren vor, bei dem eine Aromatenfraktion gewonnen und diese durch Hydrierung in eine Cycloparaffine enthaltende Fraktion umgewandelt wird. Letztere wird als Recyclestrom zu dem Olefinsynthesereaktor zurückgeführt und in diesem teilweise zu Olefinen, darunter Propylen, umgesetzt. Auf diese Weise wird bereits eine Steigerung der Propylenausbeute erreicht, allerdings bleiben immer noch Reaktivkomponenten, insbesondere C₅₊-Olefine, für die Propylenbildung ungenutzt und werden beispielweise dem MTP-Benzin zugeschlagen. Ein anderer Teil der C₅₊-Olefine wird über Spülströme dem Verfahren entzogen, deren eigentliche Aufgabe es ist, eine Anreicherung der C₅₊-Paraffine im MTP-Verfahren zu verhindern.

Trotz der beschriebenen Verfahrensmodifikationen, die auf eine teilweise Umwandlung der schweren Primärprodukte des MTP-Verfahrens zu Propylen abzielen, besteht vor dem Hintergrund der oben wiedergegebenen ökonomischen Überlegungen klarer Bedarf an einer weiteren Steigerung der Propylenausbeute.

### Beschreibung der Erfindung

Vor diesem Hintergrund besteht die Aufgabe der vorliegenden Erfindung darin, ein Verfahren und eine Anlage bereitzustellen, bei denen durch verbesserte Nutzung und Rückführung der Nebenprodukte der Olefinsynthesereaktion aus Oxygenaten die Propylenausbeute deutlich vergrößert werden kann, wobei auch die leichten Nebenprodukte für die Propylenproduktion genutzt werden sollen.

Diese Aufgabe wird durch ein Verfahren mit den Merkmalen des Anspruchs 1 und durch eine Anlage mit den Merkmalen des Anspruchs 9 gelöst:

### Erfindungsgemäßes Verfahren:

Verfahren zur Herstellung von Olefinen, insbesondere von Propylen, aus Oxygenaten, umfassend die folgenden Schritte:
(a) heterogen-katalysierte Umsetzung eines Wasserdampf und Oxygenate, wie Methanol und/oderDimethylether, umfassenden Eduktgemischs unter Oxygenatumsetzungsbedingungen in einem Olefinsynthesereaktor zu einem Propylen, andere Olefine, Paraffine und Aromaten enthaltenden Primärprodukt,
(b) Auftrennung des Primärprodukts mittels physikalischer Trennverfahren in eine C₅₋-Fraktion, eine C₅₊-Fraktion und eine wässrige, nicht umgesetzte Oxygenate enthaltende Phase,
(c) Auftrennung der G₅₊-Fraktion durch mehrstufige Destillation, umfassend einen Entbutaner, wobei mindestens ein Olefine enthaltender Rückführstrom erhalten wird, der zum Olefinsynthesereaktor zurückgeführt wird, und wobei ein Kohlenwasserstoffe enthaltender Benzinstrom gewonnen wird, der als Nebenprodukt aus dem Verfahren ausgeleitet wird,
(d) Verdichtung der C₅₋-Fraktion in einem ersten Verdichter und Auftrennung der verdichteten C₅₋-Fraktion in einer ersten Phasentrennstufe in ein erstes gasförmiges Verdichterprodukt und ein erstes flüssiges Verdichterprodukt,
(e) Zuführung des ersten flüssigen Verdichterprodukts zu dem Entbutaner, wobei als Kopfprodukt des Entbutaners eine C₄₋-Fraktion erhalten wird, die einem Entpropaner zugeführt wird,
(f) Zuführung des ersten gasförmigen Verdichterprodukts zu dem Entpropaner, wobei als Kopfprodukt des Entpropaners eine C₃₋-Fraktion und als Sumpfprodukt eine C4-Fraktion erhalten werden, wobei die C₄-Fraktion mindestens teilweise zum Olefinsynthesereaktor zurückgeführt wird,
(g) Zuführung der C₃₋-Fraktion zu einem Entethaner, wobei als Kopfprodukt des Entethaners eine C₂₋-Fraktion und als Sumpfprodukt eine C₃-Fraktion erhalten werden,
(h) Auftrennung der C₃-Fraktion mittels physikalischer Trennverfahren in eine Propylen umfassende Fraktion, die als Zielprodukt aus dem Verfahren ausgeleitet wird, und in eine Propan umfassende Fraktion,
**dadurch gekennzeichnet, dass**
(i) die Propan umfassende Fraktion einer Propandehydrierungsstufe zugeführt wird, in der unter Propandehydrierungsbedingungen Propan mindestens teilweise zu Propylen dehydriert wird, wobei das in der Propandehydrierungsstufe erzeugte Propylen dem Zielprodukt zugeführt wird.

### Erfindungsgemäße Anlage:

Anlage zur Herstellung von Olefinen aus Oxygenaten, insbesondere zur Durchführung des Verfahrens nach einem der vorhergehenden Ansprüche, umfassend folgende miteinander in Fluidverbindung stehenden Anlagenteile:
- wenigstens einen Olefinsynthesereaktor zur heterogen-katalysierten Umsetzung von wenigstens einem Oxygenat zu Propylen, andere Olefine, Paraffine und Aromaten enthaltenden Primärprodukt,
- eine Trennvorrichtung zur Auftrennung des Primärprodukts mittels physikalischer Trennverfahren in eine C₅₋-Fraktion, eine C₅₊-Fraktion und eine wässrige, nicht umgesetzte Oxygenate enthaltende Phase,
- eine mehrstufige Destillationsvorrichtung zur Auftrennung der C₅₊-Fraktion, umfassend einen Entbutaner sowie mindestens eine Leitung zur Rückführung eines Olefine enthaltenden Rückführstroms zum Olefinsynthesereaktor und eine Leitung zur Abführung eines Kohlenwasserstoffe enthaltenden Benzinstroms,
- einen ersten Verdichter und eine mit diesem verbundene erste Phasentrennstufe zur Verdichtung der C₅₋-Fraktion und Auftrennung der verdichteten C₅₋-Fraktion in ein erstes gasförmiges Verdichterprodukt und ein erstes flüssiges Verdichterprodukt,
- eine Leitung zur Zuführung des ersten flüssigen Verdichterprodukts zu dem Entbutaner,
- einen Entpropaner,
- eine Leitung zur Zuführung des Kopfprodukts des Entbutaners zu dem Entpropaner,
- eine Leitung zur Zuführung des ersten gasförmigen Verdichterprodukts zu dem Entpropaner,
- eine Leitung zur mindestens teilweise Rückführung des Sumpfprodukts des Entpropaners zum Olefinsynthesereaktor,
- einen Entethaner,
- eine Leitung zur Zuführung des Kopfprodukts des Entpropaners zum Entethaner,
- eine Leitung zur Abführung einer C₂₋-Fraktion als Kopfprodukt des Entethaners,
- eine Trennvorrichtung zur Auftrennung der C₃-Fraktion mittels physikalischer Trennverfahren in eine Propylen umfassende Fraktion und in eine Propan umfassende Fraktion,
- eine Leitung zur Ausleitung der Propylen umfassenden Fraktion als Zielprodukt,
**gekennzeichnet durch**
- eine Propandehydrierungsstufe,
- eine Leitung zur Zuführung der Propan umfassenden Fraktion zu der Propandehydrierungsstufe,
- eine Leitung zur Abführung des in der Propandehydrierungsstufe erzeugten Propylens.

Die für die Umsetzung von Oxygenaten zu Olefinprodukten erforderlichen Oxygenatumsetzungsbedingungen und die zur Dehydrierung von Propan zu Propylen erforderlichen Dehydrierungsbedingungen sind dem Fachmann bekannt. Dies betrifft insbesondere, aber nicht ausschließlich, Verfahrensbedingungen wie Temperaturen, Drücke, geeignete Raumgeschwindigkeiten und Katalysatoren. Notwendige Anpassungen dieser Bedingungen an die jeweiligen Betriebserfordemisse wird er auf der Grundlage von Routineversuchen vornehmen. Dabei können die nachfolgend offenbarten, spezifischen Reaktionsbedingungen als Orientierung dienen, sie sind aber in Bezug auf den Umfang der Erfindung nicht einschränkend zu verstehen.

Thermische Trennverfahren im Sinne der Erfindung sind alle Trennverfahren, die auf der Einstellung eines thermodynamischen Phasengleichgewichtes beruhen. Bevorzugt sind die die Destillation oder Rektifikation. Grundsätzlich ist aber auch der Einsatz anderer thermischer Trennverfahren denkbar, beispielsweise der Extraktion oder Extraktivdestillation.

Unter Fluidverbindung zwischen zwei Bereichen oder Anlagenteilen wird dabei jegliche Art von Verbindung verstanden, die es ermöglicht, dass ein Fluid, beispielsweise ein Reaktionsprodukt oder eine Kohlenwasserstofffraktion, von dem einen zu dem anderen der beiden Bereiche strömen kann, unbeachtlich etwaiger zwischengeschalteter Bereiche, Bauteile oder benötigter Fördermittel.

Unter Oxygenaten werden grundsätzlich alle sauerstoffhaltigen Kohlenwasserstoffverbindungen verstanden, die sich unter Oxygenatumsetzungsbedingungen zu Olefinen, insbesondere zu kurzkettigen Olefinen wie Propylen, und weiteren Kohlenwasserstoffprodukten umsetzen lassen.

Als kurzkettige Olefine werden im Rahmen der vorliegenden Erfindung insbesondere die Olefine verstanden, die bei Umgebungsbedingungen gasförmig vorliegen, beispielsweise Ethylen, Propylen sowie die isomeren Butene 1-Buten, cis-2-Buten, trans-2-Buten, iso-Buten.

Zur Bezeichnung von Kohlenwasserstofffraktionen wird folgende Nomenklatur verwendet: "Cₙ-Fraktion" bezeichnet eine Kohlenwasserstofffraktion, die hauptsächlich Kohlenwasserstoffe der C-Kettenlänge n, also mit n C-Atomen, enthält. "Cₙ₋-Fraktion" bezeichnet eine Kohlenwasserstofffraktion, die Kohlenwasserstoffe der C-Kettenlänge n sowie mit niedrigeren C-Kettenlängen enthält. "Cₙ₊-Fraktion" bezeichnet eine Kohlenwasserstofffraktion, die Kohlenwasserstoffe der C-Kettenlänge n sowie mit höheren C-Kettenlängen enthält. Aufgrund der verwendeten physikalischen Trennverfahren, beispielsweise der Destillation, ist die Auftrennung hinsichtlich der C-Kettenlänge nicht in der Weise zu verstehen, dass Kohlenwasserstoffe mit anderer Kettenlänge rigoros ausgeschlossen werden. So enthält eine Cₙ₋-Fraktion je nach Verfahrensbedingungen des Trennverfahrens immer noch geringe Mengen von Kohlenwasserstoffen mit einer C-Zahl größer als n.

Unter Enthexaner, Entbutaner, Entpropaner, Entethaner wird eine nach einem physikalischen Trennprinzip arbeitende Trennvorrichtung verstanden, bei der eine leichte Kohlenwasserstoff umfassende Fraktion, die als schwersten Bestandteil den namensgebenden Kohlenwasserstoff umfasst, von einer schwerere Kohlenwasserstoffe umfassenden Fraktion abgetrennt wird, wobei sich die Adjektive "leicht" und "schwer" auf die jeweilige . Molmasse der Kohlenwasserstoffe beziehen. In der Regel arbeiten diese Trennvorrichtungen als Destillations- oder Rektifikationskolonne und es ist der namensgebende Kohlenwasserstoff im Kopfprodukt der Kolonne enthalten.

### Bevorzugte Ausgestaltungen der Erfindung

In bevorzugter Ausgestaltung der Erfindung wird das in der Propandehydrierungsstufe erzeugte Propylen dem ersten Verdichter zugeführt. Auf diese Weise kann der bereits vorhandene Verdichter zusätzlich zur Verdichtung des Produktstroms der Propandehydrierungsstufe genutzt werden. Das in diesem Strom enthaltene Propylen somit in den Aufarbeitungsweg für die leichten Reaktionsprodukte und schließlich in den Propylen umfassenden Zielproduktstrom.

In alternativer bevorzugter Ausgestaltung der Erfindung wird das in der Propandehydrierungsstufe erzeugte Propylen dem Entbutaner zugeführt. Hierzu ist gegebenenfalls eine weitere Verdichtung des Produktstroms der Propandehydrierungsstufe in einem zweiten Verdichter erforderlich. Diese Ausgestaltung ist vorteilhaft, wenn, beispielsweise aufgrund der gewählten Verfahrensbedingungen, in der Propandehydrierungsstufe auch Kohlenwasserstoffe mit vier und ggf. mehr Kohlenstoffatomen gebildet werden. Diese können dann ohne Umweg über den ersten Verdichter direkt dem Entbutaner als dafür vorgesehene Aufarbeitungsvorrichtung zugeführt werden. Der erste Verdichter wird somit entlastet und kann kleiner dimensioniert werden.

In weiterer alternativer bevorzugter Ausgestaltung der Erfindung wird das in der Propandehydrierungsstufe erzeugte Propylen einem zweiten Verdichter und nachfolgend einer zweiten Phasentrennstufe zugeführt wird, wobei ein zweites gasförmiges Verdichterprodukt erhalten wird, das eine C₄₋-Fraktion umfasst, die dem Entpropaner zugeführt wird, und wobei ein zweites flüssiges Verdichterprodukt erhalten wird, das eine C₄₊-Fraktion umfasst, die dem Entbutaner zugeführt. Auch diese Ausgestaltung ist vorteilhaft, wenn in der Propandehydrierungsstufe auch Kohlenwasserstoffe mit vier und ggf. mehr Kohlenstoffatomen gebildet werden. Es fallen zwar Investitionskosten für einen zweiten Verdichter und eine zweiten Phasentrennstufe an, jedoch können durch die Fraktionierung des zweiten Verdichterprodukts in eine Gasphase und eine Flüssigphase diese Phasen zielgerichtet dem Entpropaner bzw. Entbutaner zugeführt werden, wodurch diese Apparate entsprechend kleiner ausgelegt werden können.

In weiterer alternativer bevorzugter Ausgestaltung der Erfindung wird das in der Propandehydrierungsstufe erzeugte Propylen einem zweiten Verdichter und nachfolgend einer zweiten Phasentrennstufe zugeführt wird, wobei ein zweites gasförmiges Verdichterprodukt erhalten wird, das eine C₃₋-Fraktion umfasst, die dem Entethaner zugeführt wird, und wobei ein zweites flüssiges Verdichterprodukt erhalten wird, das eine C₄₊-Fraktion umfasst, die dem Entbutaner zugeführt wird. Die Vorteile dieser Ausgestaltung entsprechen im Wesentlichen den der zuvor genannten Ausgestaltung; jedoch werden hier die Betriebsparameter der zweiten Phasentrennstufe so verändert, dass das zweite gasförmige Verdichterprodukt mehr leichte Kohlenwasserstoffe enthält, so dass sich die Zuführung zu dem Entethaner anstelle des Entpropaners anbietet.

Bevorzugt wird die Propan umfassende Fraktion vor der Zuführung zu der Propandehydrierungsstufe über einen Expander zur Energierückgewinnung geführt. Hierdurch kann die durch Expansion rückgewonnene Energie beispielsweise für den Antrieb eines Verdichters genutzt werden.

Ferner bevorzugt wird der das in der Propandehydrierungsstufe erzeugte Propylen enthaltende Stoffstrom so geführt, dass er seinen Wärmeinhalt mindestens teilweise durch indirekten Wärmetausch auf die Propan umfassende Fraktion überträgt, die der Propandehydrierungsstufe zugeführt wird. Auf diese Weise kann der Wärmeinhalt des die Propandehydrierungsstufe verlassenden Stoffstroms besser genutzt werden und es wird Heizenergie eingespart.

Günstig ist es, wenn die in Verfahrensschritt (b) erhaltene, wässrige, nicht umgesetzte Oxygenate enthaltende Phase durch Destillation aufgetrennt wird, wobei ein oxygenatreicher Strom erhalten wird, der zum Olefinsynthesereaktor zurückgeführt wird, und wobei ein Wasserdampf umfassender Strom erhalten wird, der mindestens teilweise der Propandehydrierungsstufe zugeführt wird. Vorteilhaft ist es dabei, dass im wässrigen Kondensat enthaltene Oxygenatanteile durch Umsetzung zu Olefinen stofflich genutzt werden können. Die Zuführung des Wasserdampf umfassenden Stroms zu der Propandehydrierungsstufe erbringt besondere Vorteile, da zum stabilen Betrieb der Propandehydrierungsstufe Verdünnungsdampf benötigt wird, dessen Erzeugung sehr energieintensiv ist.

Weiterhin hat es sich als vorteilhaft herausgestellt, bereits nach der heterogen-katalysierten Olefinsynthese eine C₅₋-Fraktion vom verbleibenden Reststrom abzutrennen. So können die besonders werthaltigen niedermolekularen Olefine, insbesondere das Propylen selbst, sofort aus dem Produktstrom der heterogen-katalysierten Umsetzung abgezogen werden, weshalb alle sich anschließenden Anlagenkomponenten für die Aufarbeitung der höherwertigen Olefine kleiner dimensioniert werden können.

Die Abtrennung erfolgt vorteilhafterweise durch eine Abkühlung, bei der die C₅₋-Fraktion gasförmig bleibt und sich aufgrund ihres Aggregatzustandes von dem verbleibenden flüssigen Reststrom trennt. Die so gewonnene Energie kann an anderer Stelle im Prozess genutzt werden.

Aus dem verbleibenden Reststrom wird zum einen eine wässrige, Oxygenate enthaltene Fraktion und zum anderen eine C₅₊-Fraktion gewonnen. Vorzugsweise wird dies durch eine einfache Phasentrennung erreicht, wodurch hohe Energiekosten, wie etwa eine Destillation sie bedingt, entfallen.

Vorteilhafterweise wird die wässrige, Oxygenate enthaltende Fraktion einer Trennoperation unterzogen, bei der die Oxygenate, also im Wesentlichen Methanol und/oder Dimethylether und das Wasser voneinander getrennt werden. Diese Trennung erfolgt bevorzugt in einer Destillation, um eine ausreichende Trennschärfe sicherzustellen. Wenigstens Teile des Wassers werden aus dem Verfahren ausgeschleust. Abgetrenntes Methanol und/oder abgetrenntes Wasser können in die heterogene-katalysierte Olefinsynthese zurückgeführt werden. Die Rückführung des Wassers erfolgt vorzugsweise als Dampf.

In einer besonders bevorzugten Ausgestaltung des Gesamtverfahrens erfolgt die Olefinsynthese in zwei Stufen, wobei in der ersten Stufe das wenigstens eine Oxygenat zuerst zu wenigstens einem korrespondierenden Ether und in der zweiten Stufe der/die Ether zu Olefinen umgesetzt wird/werden. Wird Methanol als Oxygenat verwendet, folgt zuerst eine Umsetzung des Methanols zu Dimethylether (DME) und anschließend die Umsetzung des Dimethylethers zu Propylen und anderen Olefinen, insbesondere auch zu Aromaten. Bei dieser zweistufigen Ausgestaltung empfiehlt es sich, Methanol bereits vor die erste Stufe, also vor die Umsetzung zu Dimethylether, zurückzuführen, während das dampfförmige Wasser zwischen die erste und zweite Stufe eingebracht wird, so dass in der ersten Stufe kein unnötiges Wasser eingesetzt wird, welches die Gleichgewichtsreaktion bei der Veretherung negativ beeinflusst; der Dampf steht jedoch für die Zuführung in die Olefinsynthese zur Verfügung.

Das Produkt aus der Oxygenatumsetzungsreaktion wird mittels dem Fachmann bekannten Methoden zunächst gekühlt, wobei Wasser und im Wasser lösliche Komponenten wie Oxygenate (Methanol, DME) auskondensiert werden und damit auf einfache Weise vom restlichen Kohlenwasserstoffprodukt abgetrennt werden können. Der resultierende wässrige Strom wird dann einer geeigneten Trenneinrichtung (z.B. einer Destillationskolonne) zugeführt, wobei die Oxygenate wie oben beschrieben in die erste Reaktionsstufe zurückgeführt werden. Die aus der Umsetzung der Oxygenate entstandene Wassermenge wird aus dem Prozess entfernt, während die Restmenge wie oben beschrieben vor die zweite Reaktionsstufe zurückgeführt wird, so dass sich größtenteils geschlossene Kreisläufe ergeben.

Der weitgehend wasserfreie Kohlenwasserstoffstrom nach der Kühlung wird komprimiert; es entsteht ein unter Druck stehender leichter Kohlenwasserstrom und eine flüssiger, ebenfalls unter Druck stehender schwerer Kohlenwasserstoffstrom. Um eventuell noch enthaltene leichtere Olefine sicher abzutrennen, empfiehlt es sich, eine weitere Trennstufe zu schalten, in der aus der C₅₊-Fraktion noch eventuell enthaltene C₄₋-Fraktionen entfernt werden können. Vorteilhafterweise ist diese Trennstufe eine Destillation, um eine ausreichende Trennschärfe sicherzustellen.

In besonderer Ausgestaltung der erfindungsgemäßen Anlage umfasst diese auch eine Leitung zur Zuführung des in der Propandehydrierungsstufe erzeugten Propylens zu dem ersten Verdichter. Auf diese Weise kann der bereits vorhandene Verdichter zusätzlich zur Verdichtung des Produktstroms der Propandehydrierungsstufe genutzt werden. Das in diesem Strom enthaltene Propylen somit in den Aufarbeitungsweg für die leichten Reaktionsprodukte und schließlich in den Propylen umfassenden Zielproduktstrom.

In alternativer bevorzugter Ausgestaltung der erfindungsgemäßen Anlage umfasst diese auch eine Leitung zur Zuführung des in der Propandehydrierungsstufe erzeugten Propylens zum Entbutaner. Ferner kann zusätzlich ein zweiter Verdichter zur weiteren Verdichtung des Produktstroms der Propandehydrierungsstufe umfasst werden. Diese Ausgestaltung ist vorteilhaft, wenn, beispielsweise aufgrund der gewählten Verfahrensbedingungen, in der Propandehydrierungsstufe auch Kohlenwasserstoffe mit vier und ggf. mehr Kohlenstoffatomen gebildet werden. Diese können dann ohne Umweg über den ersten Verdichter direkt dem Entbutaner als dafür vorgesehene Aufarbeitungsvorrichtung zugeführt werden. Der erste Verdichter wird somit entlastet und kann kleiner dimensioniert werden.

In weiterer alternativer bevorzugter Ausgestaltung der erfindungsgemäßen Anlage umfasst diese auch:
- einen zweiten Verdichter und eine mit diesem verbundene zweite Phasentrennstufe,
- eine Leitung zur Zuführung des in der Propandehydrierungsstufe erzeugten Propylens zum zweiten Verdichter,
- eine Leitung zur Abführung eines zweiten gasförmigen Verdichterproduktes aus der zweiten Phasentrennstufe und zur Zuführung desselben zum Entpropaner,
- eine Leitung zur Abführung eines zweiten flüssigen Verdichterproduktes aus der zweiten Phasentrennstufe und zur Zuführung desselben zum Entbutaner.
Auch diese Ausgestaltung ist vorteilhaft, wenn in der Propandehydrierungsstufe auch Kohlenwasserstoffe mit vier und ggf. mehr Kohlenstoffatomen gebildet werden. Es fallen zwar Investitionskosten für einen zweiten Verdichter und eine zweiten Phasentrennstufe an, jedoch können durch die Fraktionierung des zweiten Verdichterprodukts in eine Gasphase und eine Flüssigphase diese Phasen zielgerichtet dem Entpropaner bzw. Entbutaner zugeführt werden, wodurch diese Apparate entsprechend kleiner ausgelegt werden können.

In weiterer alternativer bevorzugter Ausgestaltung der erfindungsgemäßen Anlage umfasst diese auch:
- einen zweiten Verdichter und eine mit diesem verbundene zweite Phasentrennstufe,
- eine Leitung zur Zuführung des in der Propandehydrierungsstufe erzeugten Propylens zum zweiten Verdichter,
- eine Leitung zur Abführung eines zweiten gasförmigen Verdichterproduktes aus der zweiten Phasentrennstufe und zur Zuführung desselben zum Entethaner,
- eine Leitung zur Abführung eines zweiten flüssigen Verdichterproduktes aus der zweiten Phasentrennstufe und zur Zuführung desselben zum Entbutaner.
Die Vorteile dieser Ausgestaltung entsprechen in Wesentlichen den der zuvor genannten Ausgestaltung; jedoch werden hier die Betriebsparameter der zweiten Phasentrennstufe so verändert, dass das zweite gasförmige Verdichterprodukt mehr leichte Kohlenwasserstoffe enthält, so dass sich die Zuführung zu dem Entethaner anstelle des Entpropaners anbietet.

Bevorzugt umfasst die erfindungsgemäße Anlage auch einen der Propandehydrierungsstufe vorgeschalteten Expander. Durch diesen kann die durch Expansion rückgewonnene Energie beispielsweise für den Antrieb eines Verdichters genutzt werden.

Ferner bevorzugt umfasst die erfindungsgemäße Anlage auch einen Wärmetauscher zur mindestens teilweisen Übertragung des Wärmeinhalts des Produktstroms der Propandehydrierungsstufe auf den dieser zugeführten Eduktstrom. Auf diese Weise kann der Wärmeinhalt des die Propandehydrierungsstufe verlassenden Stoffstroms besser genutzt werden und es wird Heizenergie eingespart.

Günstig ist es, wenn die erfindungsgemäße Anlage auch umfasst:
- eine Destillationsvorrichtung zur Auftrennung der wässrigen, nicht umgesetzte Oxygenate enthaltenden Phase durch Destillation,
- eine Rückführleitung zur Rückführung des bei der Destillation erhaltenen, oxygenatreichen Stroms zum Olefinsynthesereaktor,
- eine Leitung zur Zuführung mindestens eines Teils des bei der Destillation erhaltenen, Wasserdampf umfassenden Stroms zur Propandehydrierungsstufe.

Vorteilhaft ist es dabei, dass im wässrigen Kondensat enthaltene Oxygenatanteile durch Umsetzung zu Olefinen stofflich genutzt werden können. Die Zuführung des Wasserdampf umfassenden Stroms zu der Propandehydrierungsstufe erbringt besondere Vorteile, da zum stabilen Betrieb der Propandehydrierungsstufe Verdünnungsdampf benötigt wird, dessen Erzeugung sehr energieintensiv ist.

### Ausführungs- und Zahlenbeispiele

Weitere Merkmale, Vorteile und Anwendungsmöglichkeiten der Erfindung ergeben sich auch aus der nachfolgenden Beschreibung von Ausführungs- und Zahlenbeispielen und der Zeichnungen. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbeziehung.

Es zeigen:
- Fig. 1: die schematische Darstellung eines MTP-Verfahrens und einer entsprechenden Anlage gemäß Stand der Technik,
- Fig. 2: die schematische Darstellung des erfindungsgemäßen Verfahrens bzw. der erfindungsgemäßen Anlage nach einer ersten Ausgestaltung,
- Fig. 3: die schematische Darstellung des erfindungsgemäßen Verfahrens bzw. der erfindungsgemäßen Anlage nach einer zweiten Ausgestaltung,
- Fig. 4: die schematische Darstellung des erfindungsgemäßen Verfahrens bzw. der erfindungsgemäßen Anlage nach einer dritten Ausgestaltung,
- Fig. 5: die schematische Darstellung des erfindungsgemäßen Verfahrens bzw. der erfindungsgemäßen Anlage nach einer vierten Ausgestaltung.

Der nachfolgend verwendete Begriff "Kolonne" bezieht sich grundsätzlich auf eine Destillationskolonne, soweit nicht im Einzelfall ein anderes thermisches Trennverfahren angegeben ist.

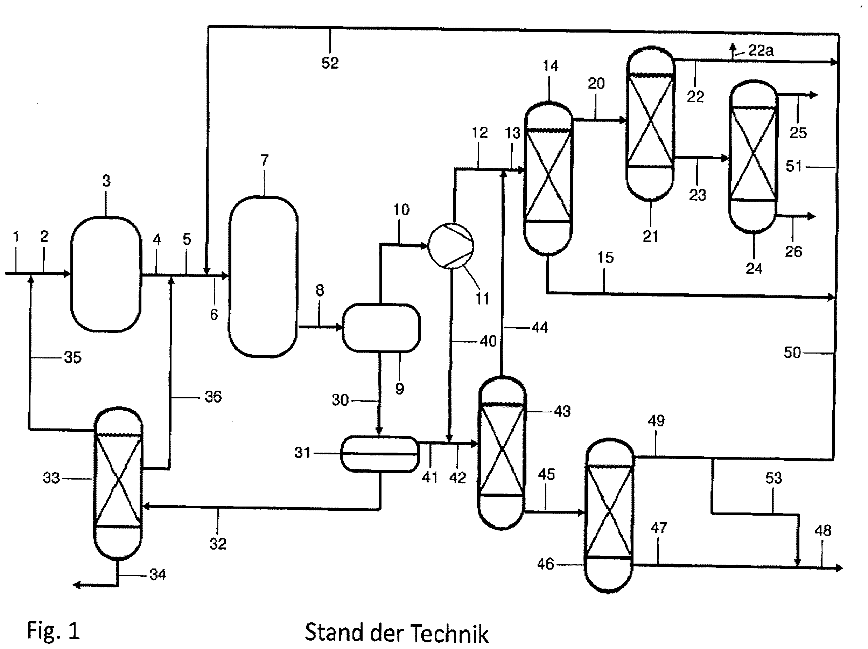

In Fig. 1 wird ein MTP-Verfahren gemäß dem Stand der Technik dargestellt. Über die Leitung 1 und 2 wird Methanol in einen Reaktor 3 eingebracht, in dem das Methanol wenigstens teilweise zu Dimethylether umgesetzt wird. Über Leitungen 4, 5 und 6 wird der Dimethylether abgezogen und einem zweiten Reaktor 7, dem Olefinsynthesereaktor, zugeführt, in dem der Dimethylether, sowie Restbestandteile an Methanol, in Anwesenheit von Dampf zu Olefinen umgesetzt werden. Der so erhaltene Olefinstrom enthält Propylen und andere Olefine, aber auch Paraffine, cyclische Olefine, cyclische Paraffine und Aromaten.

Der erhaltene Produktstrom wird über Leitung 8 in die Kühlvorrichtung 9 eingebracht. Dort trennt sich eine gasförmige von einer flüssigen Phase. Die gasförmige Phase enthält die C₅₋-Fraktion und wird über Leitung 10 einem ersten Verdichter 11 zugeführt, dem sich eine erste Phasentrennstufe anschließt (nicht bildlich gezeigt). Die in dem ersten Verdichter 11 und der ersten Phasentrennstufe entstehende gasförmige Fraktion wird über die Leitungen 12 und 13 einer Destillationskolonne 14, dem Entpropaner, zugeführt. In dieser Destillationskolonne 14 wird die C₃₋-Fraktion von der C₄₊-Fraktion getrennt.

Die C₃₋-Fraktion wird über Leitung 20 einer Kolonne 21, dem Entethaner, zugeführt, in der die C₂₋-Fraktion über Kopf abgezogen wird. Die C₂₋-Fraktion gelangt zum größten Teil über Leitung 22 und Leitung 52 zurück in Leitung 5 und kann von dort über Leitung 6 in den Olefinsynthesereaktor 7 geführt werden, so dass hier durch Olefin-Metathese zumindest teilweise das gewünschte Produkt Propylen erzeugt wird. Um eine Anreicherung von inerten leichten gasförmigen Komponenten wie Methan oder Kohlenoxiden im Kreislauf zu vermeiden, kann eine kleine Teilmenge des Stroms aus Leitung 22 über Leitung 22a als Spülstrom (Purge) aus dem System entfernt werden. Weiterhin wird aus der Kolonne 21 über Leitung 23 die C₃-Fraktion abgezogen und einer Kolonne 24, der Trennvorrichtung zur Auftrennung der C₃-Fraktion zugeführt, die auch als C₃-Splitter bezeichnet wird. In dieser Kolonne 24 wird über Kopf das erwünschte Zielprodukt Propylen abdestilliert und über Leitung 25 abgezogen, während im Sumpf Propan verbleibt und über Leitung 26 abgezogen wird.

Das Sumpfprodukt der Kolonne 14 wird als C₄-Fraktion über Leitung 15 aus der Kolonne 14 abgezogen und über die Leitung 51 und 52 ebenfalls vor die Umsetzung der Oxygenate zu Olefinen in Leitung 5 zurückgeführt, um durch Olefin-Metathese die Ausbeute an Propylen weiter zu steigern. Um eine Anreicherung von Butan (einer für die Umsetzung im Reaktor inerten Komponente) im Kreislauf zu vermeiden, kann eine kleine Teilmenge des Stroms aus Leitung 15 über eine nicht gezeigte Leitung als Purge aus dem System entfernt werden.

Die in dem Kühler 9 erhaltene, flüssige Fraktion wird über Leitung 30 einem Separator 31 zugeführt. Die in dem Separator 31 abgetrennte wässrige Phase enthält auch Oxygenate (bei der Verwendung von Methanol als Edukt vor allem Methanol, aber auch Dimethylether) und wird über Leitung 32 einer Destillationskolonne 33 zugeführt.

Aus dem Sumpf der Destillationskolonne 33 wird über Leitung 34 Wasser ausgeschleust. Weiterhin wird über Leitung 36 Dampf aus der Destillationskolonne 33 abgezogen und in Leitung 4 eingespeist, von wo der Dampf über Leitung 5 und Leitung 6 in den Reaktor 7 gelangt, wo er als Verdünnungsmedium für die Umsetzung des Dimethylethers zu Olefinen verwendet wird.

Das Kopfprodukt der Destillationskolonne 33, das wenigstens ein Oxygenat, vorzugsweise Methanol, enthält, wird über Leitung 35 in die Leitung 1 eingespeist und gelangt so über Leitung 2 in den Reaktor 3. Die Destillationskolonne 33 kann auch als Kombination mehrerer hintereinander geschalter Kolonnen ausgestaltet werden (nicht bildlich gezeigt). Wird als Edukt Methanol verwendet, so wird so rückgewonnenes Methanol zusammen mit dem als Edukt eingespeisten Methanol zu Dimethylether umgesetzt. Alternativ kann das Oxygenat auch direkt mit dem Wasserdampf über Leitung 36 zurück in den Reaktor 7 geleitet werden. Dies empfiehlt sich insbesondere dann, wenn als Oxygenat neben Methanol auch signifikante Mengen an Dimethylether vorhanden sind.

Die aus dem Separator abgezogene organische Phase enthält die C₅₊-Fraktion, welche über Leitung 41 ausgeschleust und über eine Pumpe (nicht bildlich gezeigt) weitergeleitet wird. Dieser C₅₊-Fraktion wird dann auch die aus dem ersten Verdichter 11 bei 15 bis 25 bar gewonnene flüssige Fraktion über Leitung 40 zugemischt. Die so vereinigten Ströme werden über Leitung 42 in eine Kolonne 43, den Entbutaner, eingebracht. Über Kopf wird aus der Kolonne 43 über Leitung 44 die C₄₋-Fraktion in die Leitung 12 geführt, von wo sie zusammen mit dem gasförmigen Teil aus dem Verdichter 11 über Leitung 13 in die Kolonne 14 gespeist wird.

Im vorliegenden Beispiel wird über Leitung 45 das Sumpfprodukt der Kolonne 43, das die C₅₊-Fraktion enthält, in die Kolonne 46, den Enthexaner, geführt. Aus der Kolonne 46 wird dann über den Sumpf die schwere, C₇₊-Kohlenwasserstoffe umfassende Fraktion in die Leitung 47 und 48 abgezogen. Es sind im Stand der Technik aber auch andere Aufarbeitungsmöglichkeiten für die C5+-Faktion bekannt. Diese werden hier nicht erörtert, da die Wirkungsweise der Erfindung durch sie nicht berührt wird.

Über Kopf der Kolonne 46 wird über die Leitungen 49, 50, 51 und 52 die erhaltene C₇₋-Fraktion, die auch C₅/C₆-Kohlenwasserstoffe umfasst, recycelt, indem sie in die Leitung 5 zurückgeführt wird. Teile der C₇₋-Fraktion werden über Leitung 53 der Leitung 47 zugeführt und über Leitung 48 aus dem Prozess ausgeschleust (Purge). Der aus Leitung 48 den Prozess verlassene Strom stellt das MTP-Benzin dar, das als Nebenprodukt aus dem Verfahren ausgeleitet wird.

Die Wirkungsweise der Erfindung ist aber grundsätzlich von der Weiterverarbeitung des über Leitung 45 ausgeleiteten Sumpfprodukts der Kolonne 43 unabhängig.

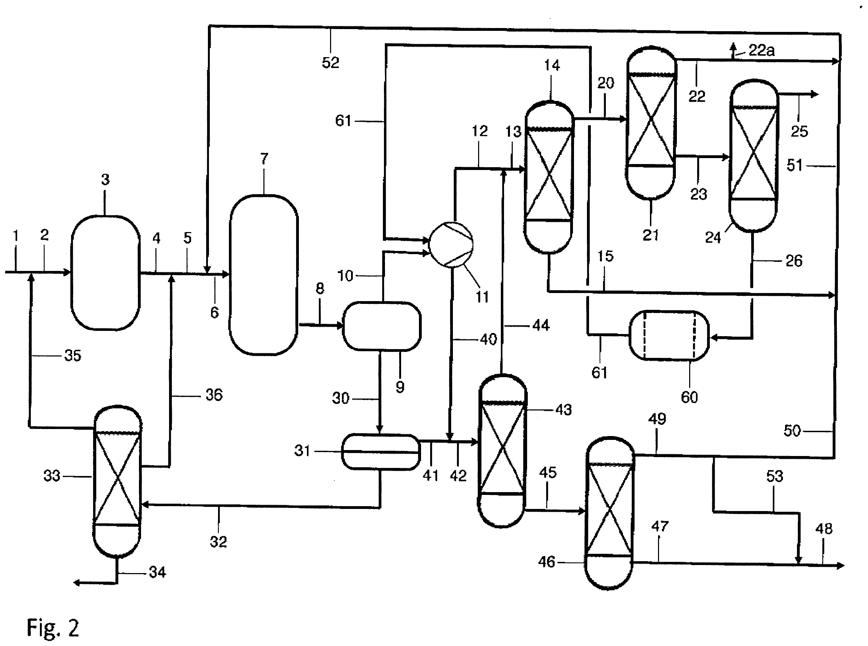

In Fig. 2 ist schematisch der Ablauf des erfindungsgemäßen Verfahrens bzw. der Aufbau einer erfindungsgemäßen Anlage nach einer ersten Ausgestaltung dargestellt. Das erfindungsgemäße Verfahren bzw. die erfindungsgemäßen Anlage entspricht bis zu der Anlagenkomponente 53 dem bereits aus dem Stand der Technik bekannten Verfahren. Das Propan enthaltende Sumpfprodukt der Kolonne 24 wird über Leitung 26 der Propandehydrierungsstufe (PDH) 60 zugeführt. Da die Propandehydrierungsstufe üblicherweise unter einem deutlich geringeren Druck arbeitet als die Kolonne 24 zur Abtrennung des Propans vom Propylen und der Produktstrom aus der Propandehydrierungsstufe zur Einbindung in den Gesamtprozess wieder verdichtet werden muss, kann vorteilhaft ein bildlich nicht dargestellter Expander zur Energierückgewinnung zwischen die Kolonne 24 und die Propandehydrierungsstufe geschaltet werden, wodurch die durch Expansion rückgewonnene für den Antrieb eines Verdichters genutzt werden kann.

Mittels eines nicht bildlich dargestellten Wärmetauschers wird ein Teil des Wärmeinhalts des die Propandehydrierungsstufe über Leitung 61 verlassenden Produktstroms auf den in Leitung 26 geführten Stoffstrom übertragen. Um sinnvolle Umsatzgrade in der Propandehydrierungsstufe zu erreichen, ist allerdings in der Regel noch eine weitere Temperaturerhöhung in einer nicht bildlich dargestellten Heizvorrichtung erforderlich.

Grundsätzlich können unterschiedliche, an sich bekannte Verfahren zur Propandehydrierung eingesetzt werden. Die spezifischen Verfahrensbedingungen ergeben sich daher aus der Art des verwendeten Verfahrens zur Propandehydrierung. Exemplarisch, aber nicht einschränkend im Sinne der Erfindung, kann das in der Offenlegungsschrift DE 199 53 641 A1 beschriebene Propandehydrierungsverfahren verwendet werden. Hierbei wird kömiger Dehydrierungskatalysator als Schüttung in von außen beheizten Röhren angeordnet. Die Kohlenwasserstoffe werden durch die Katalysatorschüttung geleitet, wobei die Temperaturen in der Katalysatorschüttung im Bereich von 400 bis 750 °C liegen. Die Röhren weisen im Innern einen katalysatorfreien, von einem Innenrohr umgebenden Raum auf, der von einem Heizfluid durchströmt wird. Vorzugsweise hat das Heizfluid beim Eintritt in den katalysatorfreien Raum Temperaturen im Bereich von 500 bis 800 °C. Als Heizfluid kann z. B. ein Produktstrom aus dem MTP-Prozess selber verwendet werden.

Der Dehydrierungskatalysator, der den Raum zwischen dem Innenrohr und dem Außenrohr ausfüllt, besteht aus Gamma-Al₂O₃ mit 0,6 Gew.-% Platin (Pt), 8 Gew.-% Kaliumoxid (K₂O) und 2 Gew.-% Zinn (Sn).

Dem in die Propandehydrierungsstufe über Leitung 26 eintretenden Stoffstrom wird Wasserdampf im Molverhältnis Propan / Dampf von 3,0 zugegeben. Das Einsatzgemisch tritt vorgewärmt auf 550 °C in die Katalysatorschüttung ein. Die Raumgeschwindigkeit, bezogen auf Propan im Einsatzgemisch, beträgt 2,0 m³/(h m³_{Kat}). Unter diesen Bedingungen beträgt der Propanumsatz in Abhängigkeit von der Laufzeit des Katalysators typischerweise zwischen 50 und 70 % und die Propylenausbeute typischerweise zwischen 40 und 65 %, bezogen auf mol C.

Der Produktstrom aus der Propandehydrierungsstufe, der das zusätzlich gebildete Propylen, Wasserstoff als Koppelprodukt und nicht umgesetztes Propan enthält, wird über Leitung 61 aus der Propandehydrierungsstufe ausgeleitet und nach indirektem Wärmetausch mit dem in Leitung 26 zu der Propandehydrierungsstufe geführten Stoffstrom in einem nicht bildlich dargestellten Wärmetauscher, sowie nach weiterer Abkühlung in einer ebenfalls nicht bildlich dargestellten Kühlvorrichtung, dem ersten Verdichter 11 zugeführt. Auf diese Weise wird das zusätzlich gebildete Propylen in der bereits vorhandenen Produktaufarbeitung gemeinsam mit dem im Olefinsynthesereaktor erzeugten Propylen gewonnen und über Leitung 25 aus dem Verfahren ausgeleitet. Der ebenfalls gebildete Wasserstoff verlässt über Leitung 22 und über Leitung 22a als Spülstrom (Purge) das Verfahren. Der Spülstrom kann als Heizgas genutzt werden, wobei der enthaltene Wasserstoff den Heizwert weiter erhöht.

In einer alternativen Ausgestaltung wird ein Teilstrom des Wasserdampfes aus Leitung 36 auch in die Propandehydrierungsstufe geleitet, um günstigere Reaktionsbedingungen (Wärmesenke, Partialdruck, ...) zu erhalten. In diesem Falle würde der Produktstrom der Propandehydrierungsstufe, nach einem optionalen Wärmeaustausch mit dem in die Propandehydrierungsstufe über Leitung 26 eintretenden Stoffstrom, direkt dem Kühler 9 erfolgen, so dass das Wasser wieder abgeschieden und weiter verwendet werden kann, während das dann weitgehende trockene Gase der weiteren Aufarbeitung zugeführt werden kann.

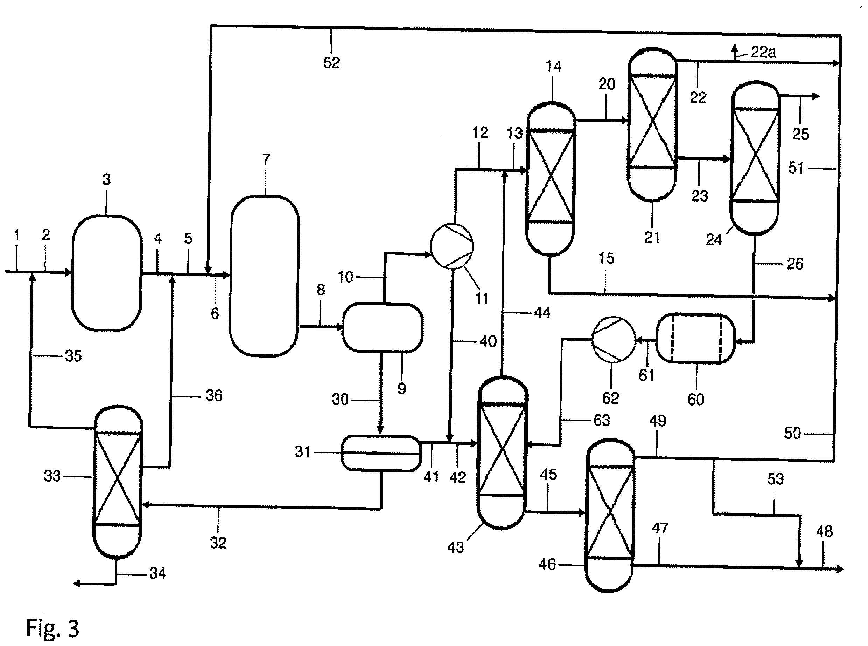

In der in Fig. 3 dargestellten Ausgestaltung des erfindungsgemäßen Verfahrens bzw. der erfindungsgemäßen Anlage ergibt sich als Unterschied zu der in Fig. 2 gezeigten Ausgestaltung, dass der die Propandehydrierungsstufe über Leitung 61 verlassende Stoffstrom einem zweiten Verdichter 62 zugeführt wird. Der verdichtete Stoffstrom wird über Leitung 63 der Kolonne 43, dem Entbutaner, zugeführt. Diese Ausgestaltung ist vorteilhaft, wenn in der Propandehydrierungsstufe auch Kohlenwasserstoffe mit vier und ggf. mehr Kohlenstoffatomen gebildet werden. Diese können dann ohne Umweg über den ersten Verdichter direkt dem Entbutaner als dafür vorgesehene Aufarbeitungsvorrichtung zugeführt werden. Der erste Verdichter wird somit entlastet und kann kleiner dimensioniert werden.

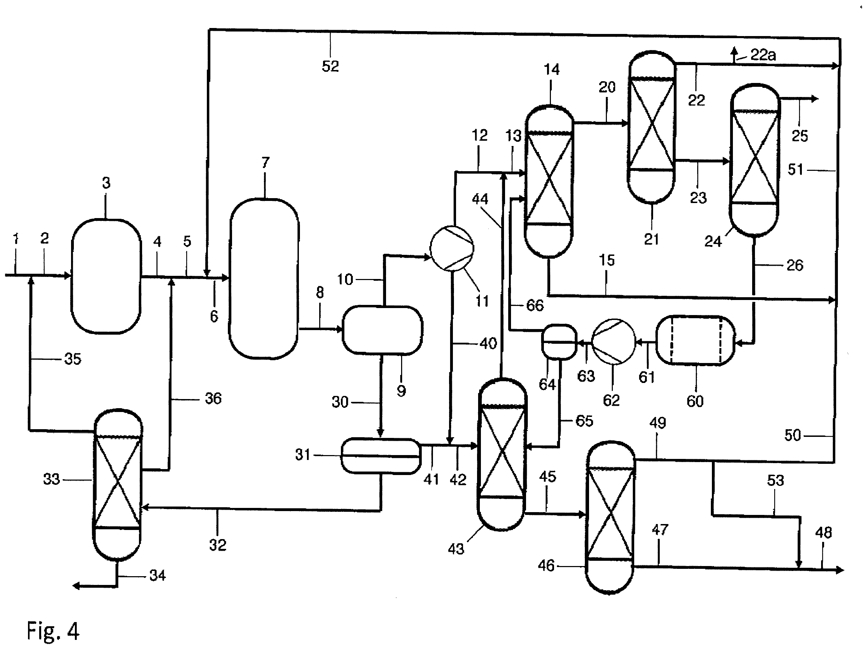

In der in Fig. 4 dargestellten Ausgestaltung des erfindungsgemäßen Verfahrens bzw. der erfindungsgemäßen Anlage ergibt sich als Unterschied zu der in Fig. 3 gezeigten Ausgestaltung, dass der den zweiten Verdichter über Leitung 63 verlassende Stoffstrom zunächst einer zweiten Phasentrennstufe 64 zugeführt wird, wobei ein zweites gasförmiges Verdichterprodukt erhalten wird, das eine C₄₋-Fraktion umfasst und über Leitung 66 dem Entpropaner 14 zugeführt wird. Das ebenfalls erhaltene zweite flüssige Verdichterprodukt enthält eine C₄₊-Fraktion und wird über Leitung 65 dem Entbutaner 43 zugeführt. Auch diese Ausgestaltung ist vorteilhaft, wenn in der Propandehydrierungsstufe auch Kohlenwasserstoffe mit vier und ggf. mehr Kohlenstoffatomen gebildet werden. Es fallen zwar Investitionskosten für einen zweiten Verdichter und eine zweiten Phasentrennstufe an, jedoch können durch die Fraktionierung des zweiten Verdichterprodukts in eine Gasphase und eine Flüssigphase diese Phasen zielgerichtet dem Entpropaner bzw. Entbutaner zugeführt werden, wodurch diese Apparate entsprechend kleiner ausgelegt werden können.

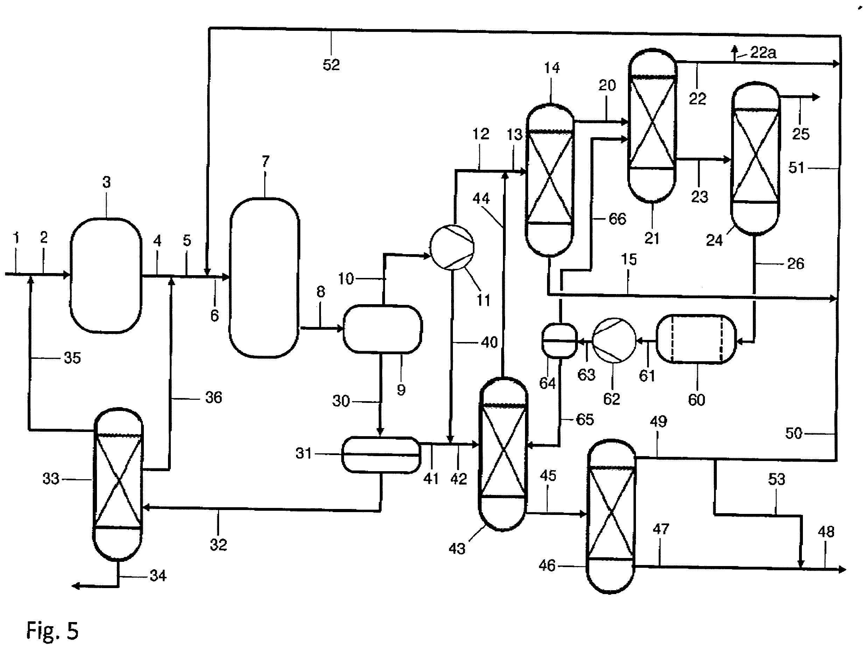

In der in Fig. 5 dargestellten Ausgestaltung des erfindungsgemäßen Verfahrens bzw. der erfindungsgemäßen Anlage ergibt sich als Unterschied zu der in Fig. 4 gezeigten Ausgestaltung, dass das zweite gasförmige Verdichterprodukt über Leitung 66 dem Entethaner 21 zugeführt wird. Die Vorteile dieser Ausgestaltung entsprechen im Wesentlichen den der zuvor genannten Ausgestaltung; jedoch werden hier die Betriebsparameter der zweiten Phasentrennstufe so verändert, dass das zweite gasförmige Verdichterprodukt mehr leichte Kohlenwasserstoffe enthält, so dass sich die Zuführung zu dem Entethaner anstelle des Entpropaners anbietet.

### Zahlenbeispiel

| **Betriebsfall** | **Anteil Propylen an C3-Produkt** | **Propylen Selekti-vität (nur MTP)** | **Propylen Selekti-vität (MTP + PDH)** | **Differenz** |
|---|---|---|---|---|
| 1 | 86,4% | 53,8% | 61,0% | 7,2% |
| 2 | 89,0% | 58,1% | 64,2% | 6,1% |
| 3 | 93,2% | 59,4 % | 63,1% | 3,7% |
| 4 | 93,9 % | 64,8% | 68,4 % | 3.6 % |
| 5 | 95,3 % | 65,1 % | 67,8 % | 2.7 % |

Es wurden Simulationsrechnungen durchgeführt, die ein MTP-Verfahren nach dem Stand der Technik abbilden. Die Berechnungen zeigen, dass eine zusätzliche Propandehydrierungsstufe die Propylen-Selektivität zwischen ca. 2,7 % und 7,2 % erhöht, s. Tabelle. Die Unterschiede ergeben sich dabei durch die Betrachtung verschiedener Betriebsfälle des MTP-Verfahrens.

### Gewerbliche Anwendbarkeit

Mit der Erfindung wird eine Verbesserung des MTP-Verfahrens zur Verfügung gestellt, die es ermöglicht, das ansonsten dem Heizgas zugeschlagene oder über Spülströme dem Verfahren entzogene Propan zumindest teilweise in das Zielprodukt Propylen umzuwandeln.

### Bezugszeichenliste

- 1, 2: Leitung
- 3: DME-Reaktor
- 4 - 6: Leitung
- 7: Olefinsynthesereaktor
- 8: Leitung
- 9: Kühler
- 10: Leitung
- 11: erster Verdichter mit erster Phasentrennstufe
- 12, 13: Leitung
- 14: Destillationskolonne
- 15: Leitung
- 20: Leitung
- 21: Destillationskolonne
- 22, 22a, 23: Leitung
- 24: Destillationskolonne
- 25, 26: Leitung
- 30: Leitung
- 31: Separator
- 32: Leitung
- 33: Destillationskolonne
- 34 - 36: Leitung
- 40 - 42: Leitung
- 43: Destillationskolonne
- 44, 45: Leitung
- 46: Destillationskolonne
- 47 - 53: Leitung
- 60: Propandehydrierungsstufe
- 61: Leitung
- 62: zweiter Verdichter
- 63: Leitung
- 64: zweite Phasentrennstufe
- 65, 66: Leitung

## Patentansprüche

1. Verfahren zur Herstellung von Olefinen, insbesondere von Propylen, aus Oxygenaten, umfassend die folgenden Schritte:
(a) heterogen-katalysierte Umsetzung eines Wasserdampf und Oxygenate, wie Methanol und/oder Dimethylether, umfassenden Eduktgemischs unter Oxygenatumsetzungsbedingungen in einem Olefinsynthesereaktor zu einem Propylen, andere Olefine, Paraffine und Aromaten enthaltenden Primärprodukt,
(b) Auftrennung des Primärprodukts mittels physikalischer Trennverfahren in eine C₅₋-Fraktion, eine C₅₊-Fraktion und eine wässrige, nicht umgesetzte Oxygenate enthaltende Phase,
(c) Auftrennung der C₅₊-Fraktion durch mehrstufige Destillation, umfassend einen Entbutaner, wobei mindestens ein Olefine enthaltender Rückführstrom erhalten wird, der zum Olefinsynthesereaktor zurückgeführt wird, und wobei ein Kohlenwasserstoffe enthaltender Benzinstrom gewonnen wird, der als Nebenprodukt aus dem Verfahren ausgeleitet wird,
(d) Verdichtung der C₅₋-Fraktion in einem ersten Verdichter und Auftrennung der verdichteten C₅₋-Fraktion in einer ersten Phasentrennstufe in ein erstes gasförmiges Verdichterprodukt und ein erstes flüssiges Verdichterprodukt,
(e) Zuführung des ersten flüssigen Verdichterprodukts zu dem Entbutaner, wobei als Kopfprodukt des Entbutaners eine C₄₋-Fraktion erhalten wird, die einem Entpropaner zugeführt wird,
(f) Zuführung des ersten gasförmigen Verdichterprodukts zu dem Entpropaner, wobei als Kopfprodukt des Entpropaners eine C₃₋-Fraktion und als Sumpfprodukt eine C₄-Fraktion erhalten werden, wobei die C₄-Fraktion mindestens teilweise zum Olefinsynthesereaktor zurückgeführt wird,
(g) Zuführung der C₃₋-Fraktion zu einem Entethaner, wobei als Kopfprodukt des Entethaners eine C₂₋-Fraktion und als Sumpfprodukt eine C₃-Fraktion erhalten werden,
(h) Auftrennung der C₃-Fraktion mittels physikalischer Trennverfahren in eine Propylen umfassende Fraktion, die als Zielprodukt aus dem Verfahren ausgeleitet wird, und in eine Propan umfassende Fraktion,
**dadurch gekennzeichnet, dass**
(i) die Propan umfassende Fraktion einer Propandehydrierungsstufe zugeführt wird, in der unter Propandehydrierungsbedingungen Propan mindestens teilweise zu Propylen dehydriert wird, wobei das in der Propandehydrierungsstufe erzeugte Propylen dem Zielprodukt zugeführt wird.

2. Verfahren nach Anspruch 1, **dadurch gekennzeichnet, dass** das in der Propandehydrierungsstufe erzeugte Propylen dem ersten Verdichter zugeführt wird.

3. Verfahren nach Anspruch 1, **dadurch gekennzeichnet, dass** das in der Propandehydrierungsstufe erzeugte Propylen dem Entbutaner zugeführt wird.

4. Verfahren nach Anspruch 1, **dadurch gekennzeichnet, dass** das in der Propandehydrierungsstufe erzeugte Propylen einem zweiten Verdichter und nachfolgend einer zweiten Phasentrennstufe zugeführt wird, wobei ein zweites gasförmiges Verdichterprodukt erhalten wird, das eine C₄₋-Fraktion umfasst, die dem Entpropaner zugeführt wird, und wobei ein zweites flüssiges Verdichterprodukt erhalten wird, das eine G₄₊-Fraktion umfasst, die dem Entbutaner zugeführt wird.

5. Verfahren nach Anspruch 1, **dadurch gekennzeichnet, dass** das in der Propandehydrierungsstufe erzeugte Propylen einem zweiten Verdichter und nachfolgend einer zweiten Phasentrennstufe zugeführt wird, wobei ein zweites gasförmiges Verdichterprodukt erhalten wird, das eine C₃₋-Fraktion umfasst, die dem Entethaner zugeführt wird, und wobei ein zweites flüssiges Verdichterprodukt erhalten wird, das eine C₄₊-Fraktion umfasst, die dem Entbutaner zugeführt wird.

6. Verfahren nach einem der vorgenannten Ansprüche , **dadurch gekennzeichnet, dass** die Propan umfassende Fraktion vor der Zuführung zu der Propandehydrierungsstufe über einen Expander zur Energierückgewinnung geführt wird.

7. Verfahren nach einem der vorgenannten Ansprüche, **dadurch gekennzeichnet, dass** der das in der Propandehydrierungsstufe erzeugte Propylen enthaltende Stoffstrom seinen Wärmeinhalt mindestens teilweise durch indirekten Wärmetausch auf die Propan umfassende Fraktion überträgt, die der Propandehydrierungsstufe zugeführt wird.

8. Verfahren nach einem der vorgenannten Ansprüche, **dadurch gekennzeichnet, dass** die in Verfahrensschritt (b) erhaltene, wässrige, nicht umgesetzte Oxygenate enthaltende Phase durch Destillation aufgetrennt wird, wobei ein oxygenatreicher Strom erhalten wird, der zum Olefinsynthesereaktor zurückgeführt wird, und wobei ein Wasserdampf umfassender Strom erhalten wird, der mindestens teilweise der Propandehydrierungsstufe zugeführt wird.

9. Anlage zur Herstellung von Olefinen aus Oxygenaten, insbesondere zur Durchführung des Verfahrens nach einem der vorhergehenden Ansprüche, umfassend folgende miteinander in Fluidverbindung stehenden Anlagenteile:
- wenigstens einen Olefinsynthesereaktor zur heterogen-katalysierten Umsetzung von wenigstens einem Oxygenat zu Propylen, andere Olefine, Paraffine und Aromaten enthaltenden Primärprodukt,
- eine Trennvorrichtung zur Auftrennung des Primärprodukts mittels physikalischer Trennverfahren in eine C₅₋-Fraktion, eine C₅₊-Fraktion und eine wässrige, nicht umgesetzte Oxygenate enthaltende Phase,
- eine mehrstufige Destillationsvorrichtung zur Auftrennung der C₅₊-Fraktion, umfassend einen Entbutaner sowie mindestens eine Leitung zur Rückführung eines Olefine enthaltenden Rückführstroms zum Olefinsynthesereaktor und eine Leitung zur Abführung eines Kohlenwasserstoffe enthaltenden Benzinstroms,
- einen ersten Verdichter und eine mit diesem verbundene erste Phasentrennstufe zur Verdichtung der C₅₋-Fraktion und Auftrennung der verdichteten C₅₋-Fraktion in ein erstes gasförmiges Verdichterprodukt und ein erstes flüssiges Verdichterprodukt,
- eine Leitung zur Zuführung des ersten flüssigen Verdichterprodukts zu dem Entbutaner,
- einen Entpropaner,
- eine Leitung zur Zuführung des Kopfprodukts des Entbutaners zu dem Entpropaner,
- eine Leitung zur Zuführung des ersten gasförmigen Verdichterprodukts zu dem Entpropaner,
- eine Leitung zur mindestens teilweise Rückführung des Sumpfprodukts des Entpropaners zum Olefinsynthesereaktor,
- einen Entethaner,
- eine Leitung zur Zuführung des Kopfprodukts des Entpropaners zum Entethaner,
- eine Leitung zur Abführung einer C₂₋-Fraktion als Kopfprodukt des Entethaners,
- eine Trennvorrichtung zur Auftrennung der C₃-Fraktion mittels physikalischer Trennverfahren in eine Propylen umfassende Fraktion und in eine Propan umfassende Fraktion,
- eine Leitung zur Ausleitung der Propylen umfassenden Fraktion als Zielprodukt,
**gekennzeichnet durch**
- eine Propandehydrierungsstufe,
- eine Leitung zur Zuführung der Propan umfassenden Fraktion zu der Propandehydrie-rungsstufe,
- eine Leitung zur Abführung des in der Propandehydrierungsstufe erzeugten Propylens.

10. Anlage nach Anspruch 9, ferner umfassend eine Leitung zur Zuführung des in der Propandehydrierungsstufe erzeugten Propylens zu dem ersten Verdichter.

11. Anlage nach Anspruch 9, ferner umfassend eine Leitung zur Zuführung des in der Propandehydrierungsstufe erzeugten Propylens zum Entbutaner.

12. Anlage nach Anspruch 9, ferner umfassend:
- einen zweiten Verdichter und eine mit diesem verbundene zweite Phasentrennstufe,
- eine Leitung zur Zuführung des in der Propandehydrierungsstufe erzeugten Propylens zum zweiten Verdichter,
- eine Leitung zur Abführung eines zweiten gasförmigen Verdichterproduktes aus der zweiten Phasentrennstufe und zur Zuführung desselben zum Entpropaner,
- eine Leitung zur Abführung eines zweiten flüssigen Verdichterproduktes aus der zweiten Phasentrennstufe und zur Zuführung desselben zum Entbutaner.

13. Anlage nach Anspruch 9, ferner umfassend:
- einen zweiten Verdichter und eine mit diesem verbundene zweite Phasentrennstufe,
- eine Leitung zur Zuführung des in der Propandehydrierungsstufe erzeugten Propylens zum zweiten Verdichter,
- eine Leitung zur Abführung eines zweiten gasförmigen Verdichterproduktes aus der zweiten Phasentrennstufe und zur Zuführung desselben zum Entethaner,
- eine Leitung zur Abführung eines zweiten flüssigen Verdichterproduktes aus der zweiten Phasentrennstufe und zur Zuführung desselben zum Entbutaner.

14. Anlage nach einem der vorgenannten Ansprüche, ferner umfassend einen der Propandehydrierungsstufe vorgeschalteten Expander.

15. Anlage nach einem der vorgenannten Ansprüche, ferner umfassend einen Wärmetauscher zur mindestens teilweisen Übertragung des Wärmeinhalts des Produktstroms der Propandehydrierungsstufe auf den dieser zugeführten Eduktstrom.

16. Anlage nach einem der vorgenannten Ansprüche, ferner umfassend:
- eine Destillationsvorrichtung zur Auftrennung der wässrigen, nicht umgesetzte Oxygenate enthaltenden Phase durch Destillation,
- eine Leitung zur Rückführung des bei der Destillation erhaltenen, oxygenatreichen Stroms zum Olefinsynthesereaktor,
- eine Leitung zur Zuführung mindestens eines Teils des bei der Destillation erhaltenen, Wasserdampf umfassenden Stroms zur Propandehydrierungsstufe.

## Claims

1. A process for producing olefins, in particular propylene, from oxygenates, comprising the following steps:
(a) heterogeneously catalyzed conversion of an educt mixture comprising steam and oxygenates, such as methanol and/or dimethyl ether, under oxygenate conversion conditions in an olefin synthesis reactor to a primary product containing propylene, other olefins, paraffins and aromatics,
(b) fractionation of the primary product by means of physical separation methods into a C₅₋ fraction, a C₅₊ fraction and an aqueous phase containing non-converted oxygenates,
(c) fractionation of the C₅₊ fraction by multistage distillation, comprising a debutanizer, wherein at least one recycle stream containing olefins is obtained, which is recirculated to the olefin synthesis reactor, and wherein a gasoline stream containing hydrocarbons is obtained, which is discharged from the process as byproduct,
(d) compression of the C₅₋ fraction in a first compressor and fractionation of the compressed C₅₋ fraction in a first phase separation stage into a first gaseous compressor product and a first liquid compressor product,
(e) supplying the first liquid compressor product to the debutanizer, wherein as top product of the debutanizer a C₄₋ fraction is obtained, which is supplied to a depropanizer,
(f) supplying the first gaseous compressor product to a depropanizer, wherein as top product of the depropanizer a C₃₋ fraction and as bottom product a C₄ fraction are obtained, wherein the C₄ fraction is at least partly recirculated to the olefin synthesis reactor,
(g) supplying the C₃₋ fraction to a deethanizer, wherein as top product of the deethanizer a C₂₋ fraction and as bottom product a C₃ fraction are obtained,
(h) fractionation of the C₃ fraction by means of physical separation methods into a fraction comprising propylene, which is discharged from the process as target product, and into a fraction comprising propane,
**characterized in that**
(i) the fraction comprising propane is supplied to a propane dehydrogenation stage, in which under propane dehydrogenation conditions propane is at least partly dehydrogenated to propylene, wherein the propylene produced in the propane dehydrogenation stage is supplied to the target product.

2. The process according to claim 1, **characterized in that** the propylene produced in the propane dehydrogenation stage is supplied to the first compressor.

3. The process according to claim 1, **characterized in that** the propylene produced in the propane dehydrogenation stage is supplied to the debutanizer.

4. The process according to claim 1, **characterized in that** the propylene produced in the propane dehydrogenation stage is supplied to a second compressor and subsequently to a second phase separation stage, wherein a second gaseous compressor product is obtained, which comprises a C₄₋ fraction which is supplied to the depropanizer, and wherein a second liquid compressor product is obtained, which comprises a C₄₊ fraction which is supplied to the debutanizer.

5. The process according to claim 1, **characterized in that** the propylene produced in the propane dehydrogenation stage is supplied to a second compressor and subsequently to a second phase separation stage, wherein a second gaseous compressor product is obtained, which comprises a C₃₋ fraction which is supplied to the deethanizer, and wherein a second liquid compressor product is obtained, which comprises a C₄₊ fraction which is supplied to the debutanizer.

6. The process according to any of the aforementioned claims, **characterized in that** before being supplied to the propane dehydrogenation stage the fraction comprising propane is guided over an expander for energy recovery.

7. The process according to any of the aforementioned claims, **characterized in that** the material stream containing the propylene produced in the propane dehydrogenation stage at least partly transmits its heat content to the fraction comprising propane by indirect heat exchange, which is supplied to the propane dehydrogenation stage.

8. The process according to any of the aforementioned claims, **characterized in that** the aqueous phase containing non-converted oxygenates, which is obtained in process step (b), is fractionated by distillation, wherein a stream rich in oxygenates is obtained, which is recirculated to the olefin synthesis reactor, and wherein a stream comprising steam is obtained, which is at least partly supplied to the propane dehydrogenation stage.

9. A plant for producing olefins from oxygenates, in particular for carrying out the process according to any of the preceding claims, comprising the following plant sections which are in fluid connection with each other:
- at least one olefin synthesis reactor for the heterogeneously catalyzed conversion of at least one oxygenate to a primary product containing propylene, other olefins, paraffins and aromatics,
- a separating device for fractionating the primary product by means of physical separation methods into a C₅₋ fraction, a C₅₊ fraction and an aqueous phase containing non-converted oxygenates,
- a multistage distillation device for fractionating the C₅₊ fraction, comprising a debutanizer and at least one conduit for recirculating a recycle stream containing olefins to the olefin synthesis reactor and a conduit for discharging a gasoline stream containing hydrocarbons,
- a first compressor and a first phase separation stage connected with the same for compression of the C₅₋ fraction and fractionation of the compressed C₅₋ fraction into a first gaseous compressor product and a first liquid compressor product,
- a conduit for supplying the first liquid compressor product to the debutanizer,
- a depropanizer,
- a conduit for supplying the top product of the debutanizer to the depropanizer,
- a conduit for supplying the first gaseous compressor product to the depropanizer,
- a conduit for at least partly recirculating the bottom product of the depropanizer to the olefin synthesis reactor,
- a deethanizer,
- a conduit for supplying the top product of the depropanizer to the deethanizer,
- a conduit for discharging a C₂₋ fraction as top product of the deethanizer,
- a separating device for fractionating the C₃ fraction by means of physical separation methods into a fraction comprising propylene and into a fraction comprising propane,
- a conduit for discharging the fraction comprising propylene as target product,
**characterized by**
- a propane dehydrogenation stage,
- a conduit for supplying the fraction comprising propane to the propane dehydrogenation stage,
- a conduit for discharging the propylene produced in the propane dehydrogenation stage.

10. The plant according to claim 9, furthermore comprising a conduit for supplying the propylene produced in the propane dehydrogenation stage to the first compressor.

11. The plant according to claim 9, furthermore comprising a conduit for supplying the propylene produced in the propane dehydrogenation stage to the debutanizer.

12. The plant according to claim 9, furthermore comprising:
- a second compressor and a second phase separation stage connected with the same,
- a conduit for supplying the propylene produced in the propane dehydrogenation stage to the second compressor,
- a conduit for discharging a second gaseous compressor product from the second phase separation stage and for supplying the same to the depropanizer,
- a conduit for discharging a second liquid compressor product from the second phase separation stage and for supplying the same to the debutanizer,

13. The plant according to claim 9, furthermore comprising:
- a second compressor and a second phase separation stage connected with the same,
- a conduit for supplying the propylene produced in the propane dehydrogenation stage to the second compressor,
- a conduit for discharging a second gaseous compressor product from the second phase separation stage and for supplying the same to the deethanizer,
- a conduit for discharging a second liquid compressor product from the second phase separation stage and for supplying the same to the debutanizer,

14. The plant according to any of the aforementioned claims, furthermore comprising an expander upstream of the propane dehydrogenation stage.

15. The plant according to any of the aforementioned claims, furthermore comprising a heat exchanger for at least partly transmitting the heat content of the product stream of the propane dehydrogenation stage to the educt stream supplied to the same.

16. The plant according to any of the aforementioned claims, furthermore comprising:
- a distillation device for fractionating the aqueous phase containing non-converted oxygenates by distillation,
- a conduit for recirculating the stream rich in oxygenates, which is obtained during the distillation, to the olefin synthesis reactor,
- a conduit for supplying at least a part of the stream comprising steam, which is obtained during the distillation, to the propane dehydrogenation stage.

## Revendications

1. Procédé pour la production d'oléfines, en particulier de propylène, à partir de composés oxygénés, comprenant les étapes suivantes :
(a) la conversion catalysée par un catalyseur hétérogène d'un mélange de produits de départ comprenant de la vapeur d'eau et des composés oxygénés, tels que le méthanol et/ou l'oxyde de diméthyle, dans des conditions de conversion de composés oxygénés dans un réacteur de synthèse d'oléfines en un produit primaire contenant du propylène, d'autres oléfines, des paraffines et des composés aromatiques,
(b) le fractionnement du produit primaire au moyen de méthodes physiques de séparation en une fraction de C₅₋, une fraction de C₅₊ et une phase aqueuse contenant des composés oxygénés non convertis,
(c) le fractionnement de la fraction de C₅₊ par distillation à plusieurs étages, comprenant un débutaniseur, au moins un flux de recyclage contenant des oléfines, qui est amené à recirculer vers le réacteur de synthèse d'oléfines, étant obtenu et un flux d'essence contenant des hydrocarbures, qui est amené à sortir du procédé en tant que sous-produit, étant obtenu,
(d) la compression de la fraction de C₅₋ dans un premier compresseur et le fractionnement de la fraction de C₅₋ comprimée dans un premier étage de séparation de phases en un premier produit de compresseur gazeux et un premier produit de compresseur liquide,
(e) l'envoi du premier produit de compresseur liquide vers le débutaniseur, une fraction de C₄₋, qui est envoyée vers un dépropaniseur, étant obtenue en tant que produit de tête du débutaniseur,
(f) l'envoi du premier produit de compresseur gazeux vers un dépropaniseur, une fraction de C₃₋ étant obtenue en tant que produit de tête du dépropaniseur et une fraction de C₄ en tant que produit de fond, la fraction de C₄ étant au moins en partie amenée à recirculer vers le réacteur de synthèse d'oléfines,
(g) l'envoi de la fraction de C₃₋ vers un dééthaniseur, une fraction de C₂₋ étant obtenue en tant que produit de tête du dééthaniseur et une fraction de C₃ en tant que produit de fond,
(h) le fractionnement de la fraction de C₃ au moyen de méthodes physiques de séparation en une fraction comprenant du propylène, qui est amenée à sortir du procédé en tant que produit cible, et en une fraction comprenant du propane,
**caractérisé en ce que**
(i) la fraction comprenant du propane est envoyée vers un étage de déshydrogénation de propane, dans lequel dans des conditions de déshydrogénation de propane du propane est au moins en partie déshydrogéné en propylène, le propylène produit dans l'étage de déshydrogénation de propane étant envoyé vers le produit cible.

2. Procédé selon la revendication 1, **caractérisé en ce que** le propylène produit dans l'étage de déshydrogénation de propane est envoyé vers le premier compresseur.

3. Procédé selon la revendication 1, **caractérisé en ce que** le propylène produit dans l'étage de déshydrogénation de propane est envoyé vers le débutaniseur.

4. Procédé selon la revendication 1, **caractérisé en ce que** le propylène produit dans l'étage de déshydrogénation de propane est envoyé vers un second compresseur et par la suite vers un second étage de séparation de phases, un second produit de compresseur gazeux, qui comprend une fraction de C₄₋ qui est envoyée vers le dépropaniseur, étant obtenu et un second produit de compresseur liquide, qui comprend une fraction de C₄₊ qui est envoyée vers le débutaniseur, étant obtenu.

5. Procédé selon la revendication 1, **caractérisé en ce que** le propylène produit dans l'étage de déshydrogénation de propane est envoyé vers un second compresseur et par la suite vers un second étage de séparation de phases, un second produit de compresseur gazeux, qui comprend une fraction de C₃₋ qui est envoyée vers le dééthaniseur, étant obtenu et un second produit de compresseur liquide, qui comprend une fraction de C₄₊ qui est envoyée vers le débutaniseur, étant obtenu.

6. Procédé selon l'une quelconque des revendications susmentionnées, **caractérisé en ce qu'**avant d'être envoyée vers l'étage de déshydrogénation de propane la fraction comprenant du propane est dirigée vers un détendeur pour la récupération d'énergie.

7. Procédé selon l'une quelconque des revendications susmentionnées, **caractérisé en ce que** le flux de matière contenant le propylène produit dans l'étage de déshydrogénation de propane transmet au moins en partie son contenu thermique à la fraction comprenant du propane, qui est envoyée vers l'étage de déshydrogénation de propane, par échange indirect de chaleur.

8. Procédé selon l'une quelconque des revendications susmentionnées, **caractérisé en ce que** la phase aqueuse contenant des composés oxygénés non convertis, qui est obtenue dans l'étape de procédé (b), est fractionnée par distillation, un flux riche en composés oxygénés, qui est amené à recirculer vers le réacteur de synthèse d'oléfines, étant obtenu et un flux comprenant de la vapeur d'eau, qui est au moins en partie envoyé vers l'étage de déshydrogénation de propane, étant obtenu.

9. Installation pour la production d'oléfines à partir de composés oxygénés, en particulier pour la mise en oeuvre du procédé selon l'une quelconque des revendications précédentes, comprenant les sections d'installation suivantes qui sont en communication fluidique les unes avec les autres :
- au moins un réacteur de synthèse d'oléfines pour la conversion catalysée par un catalyseur hétérogène d'au moins un composé oxygéné en un produit primaire contenant du propylène, d'autres oléfines, des paraffines et des composés aromatiques,
- un dispositif de séparation pour le fractionnement du produit primaire au moyen de méthodes physiques de séparation en une fraction de C₅₋, une fraction de C₅₊ et une phase aqueuse contenant des composés oxygénés non convertis,
- un dispositif de distillation à plusieurs étages pour le fractionnement de la fraction de C₅₊, comprenant un débutaniseur et au moins une conduite pour la recirculation d'un flux de recyclage contenant des oléfines vers le réacteur de synthèse d'oléfines et une conduite pour la sortie d'un flux d'essence contenant des hydrocarbures,
- un premier compresseur et un premier étage de séparation de phases raccordé à celui-ci pour la compression de la fraction de C₅₋ et le fractionnement de la fraction de C₅₋ comprimée en un premier produit de compresseur gazeux et un premier produit de compresseur liquide,
- une conduite pour l'envoi du premier produit de compresseur liquide vers le débutaniseur,
- un dépropaniseur,
- une conduite pour l'envoi du produit de tête du débutaniseur vers le dépropaniseur,
- une conduite pour l'envoi du premier produit de compresseur gazeux vers le dépropaniseur,
- une conduite pour la recirculation au moins partielle du produit de fond du dépropaniseur vers le réacteur de synthèse d'oléfines,
- un dééthaniseur,
- une conduite pour l'envoi du produit de tête du dépropaniseur vers le dééthaniseur,
- une conduite pour la sortie d'une fraction de C₂₋ en tant que produit de tête du dééthaniseur,
- un dispositif de séparation pour le fractionnement de la fraction de C₃ au moyen de méthodes physiques de séparation en une fraction comprenant du propylène et en une fraction comprenant du propane,
- une conduite pour la sortie de la fraction comprenant du propylène en tant que produit cible,
**caractérisé par**
- un étage de déshydrogénation de propane,
- une conduite pour l'envoi de la fraction comprenant du propane vers l'étage de déshydrogénation de propane,
- une conduite pour la sortie du propylène produit dans l'étage de déshydrogénation de propane.

10. Installation selon la revendication 9, comprenant en outre une conduite pour l'envoi du propylène produit dans l'étage de déshydrogénation de propane vers le premier compresseur.

11. Installation selon la revendication 9, comprenant en outre une conduite pour l'envoi du propylène produit dans l'étage de déshydrogénation de propane vers le débutaniseur.

12. Installation selon la revendication 9, comprenant en outre :
- un second compresseur et un second étage de séparation de phases raccordé à celui-ci,
- une conduite pour l'envoi du propylène produit dans l'étage déshydrogénation de propane vers le second compresseur,
- une conduite pour la sortie d'un second produit de compresseur gazeux hors du second étage de séparation de phases et pour l'envoi de celui-ci vers le dépropaniseur,
- une conduite pour la sortie d'un second produit de compresseur liquide hors du second étage de séparation de phases et pour l'envoi de celui-ci vers le débutaniseur.

13. Installation selon la revendication 9, comprenant en outre :
- un second compresseur et un second étage de séparation de phases raccordé à celui-ci,
- une conduite pour l'envoi du propylène produit dans l'étage déshydrogénation de propane vers le second compresseur,
- une conduite pour la sortie d'un second produit de compresseur gazeux hors du second étage de séparation de phases et pour l'envoi de celui-ci vers le dééthaniseur,
- une conduite pour la sortie d'un second produit de compresseur liquide hors du second étage de séparation de phases et pour l'envoi de celui-ci vers le débutaniseur.

14. Installation selon l'une quelconque des revendications susmentionnées, comprenant en outre un détendeur en amont de l'étage de déshydrogénation de propane.

15. Installation selon l'une quelconque des revendications susmentionnées, comprenant en outre un échangeur de chaleur pour la transmission au moins partielle du contenu thermique du flux de produit de l'étage de déshydrogénation de propane au flux de produit de départ envoyé vers celui-ci.

16. Installation selon l'une quelconque des revendications susmentionnées, comprenant en outre :
- un dispositif de distillation pour le fractionnement par distillation de la phase aqueuse contenant des composés oxygénés non convertis,
- une conduite pour la recirculation du flux riche en composés oxygénés, qui est obtenu pendant la distillation, vers le réacteur de synthèse d'oléfines,
- une conduite pour l'envoi d'au moins une partie du flux comprenant de la vapeur d'eau, qui est obtenu pendant la distillation, vers l'étage de déshydrogénation de propane.
